# EUROPÄISCHE PATENTANMELDUNG

(11) **EP 1 291 414 A1**
(43) Veröffentlichungstag der Anmeldung: **12.03.2003**
(21) Anmeldenummer: 01121806.2
(22) Anmeldetag: 11.09.2001
(51) Int. Cl.: C12N 5/06

(54) **Ex vivo Selektion von T-Zellen mit reduzierter Alloreaktivität (vermindertem Graft-versus-Host Effekt und erhöhtem Graft-verus-Leukemia Effekt) und Gebrauch derseleben bei Knochnemarktransplantationen**

(71) Anmelder: Genethor GmbH, 13125 Berlin (DE)
(72) Erfinder: Die Erfindernennung liegt noch nicht vor
(74) Vertreter: Meyers, Hans-Wilhelm, Dr.Dipl.-Chem.

(57) **Zusammenfassung**

Die Erfindung beruht auf einer Reduktion von allologen Immunreaktionen. Bei dem Verfahren werden die regulatorischen Eigenschaften von Antigenpräsentierenden Zellen genutzt, um Graft-versus-host-Reaktionen *ex vivo* auszuschalten.

## Beschreibung

Gegenstand der vorliegenden Erfindung sind Antigen-präsentierende Zellen, Verfahren zur Herstellung von Antigen-präsentierenden Zellen, Arzneimittel enthaltend Antigen-präsentierende Zellen sowie Verwendung der Antigen-präsentierenden Zellen.

### Beschreibung

Seit knapp 10 Jahren werden für verschiedene Krankheiten und in Tiermodellen gentherapeutische Verfahren entwickelt und angewendet. Bisher sind sie noch nicht in die Routine überführt. Meist handelt es sich dabei um die Behandlung von genetisch bedingten, schweren Erkrankungen, für die andere Therapien nicht zur Verfügung stehen. Weiterhin werden Gentherapien zur Behandlung von schweren und ebenfalls nicht therapierbaren Krebserkrankungen eingesetzt.

### Allgemeines zu Transplantationen

Die Transplantation von Geweben, um kranke Organe zu ersetzen, ist heute eine wichtige medizinische Therapie. In den meisten Fällen stellt eine Reaktion des anpassungsfähigen (adaptiven) Immunsystems gegen das Transplantat die größte Bedrohung für eine erfolgreiche Behandlung dar. Reaktionen des anpassungsfähigen Immunsystems werden von Antigen-präsentierenden Zellen durch Aktivierung von T-Helfer-Lymphozyten induziert. Bei der Transfusionen von Blut, welches das erste und am häufigsten verwendete Transplantat ist, müssen die ABO und Rh Blutgruppenantigene abgeglichen werden, damit die schnelle Zerstörung unpassender Erythrozyten vermieden wird. Bei anderen Geweben müssen die sehr polymorphen Haupthistokompatibilätskomplexe (Gewebeverträglichleitskomplexe, MHC) aufeinander abgeglichen werden, da diese fast immer die Immunreaktion auslösen. Leider ist der perfekte Abgleich der MHCs außer bei Verwandten fast unmöglich, da der Grad der Übereinstimmung im MHC-Muster oft zu gering ist.

Stammzelltransplantate bei Leukämien

Leukämie ist Krebs der Blutzellen. Pro Jahr bekommen 50 von 1 Million Menschen Leukämie. Wenn sich eine Leukämie entwickelt, produziert der Körper große Mengen abnormaler Blutzellen. Bei den meisten Arten von Leukämie, sind die abnormalen Zellen weiße Blutzellen.

### Leukämiearten

Es gibt etliche Leukämiearten, welche im Wesentlichen zwei Charakteristika zeigen. Das eine Charakteristikum ist, wie schnell sich die Krankheit entwickelt und verschlimmert. Das andere wird durch den Blutzelltyp, der betroffen ist, bestimmt.

Leukämie ist entweder akut oder chronisch. Bei der akuten Leukämie sind die abnormalen Blutzellen sehr unreife Blasten, die ihre normalen Funktionen nicht ausführen können. Die Anzahl von Blasten steigt schnell an und die Leiden werden schnell schlimmer. Bei chronischer Leukämie sind einige Blasten präsent, aber im allgemeinen sind diese Zellen reifer und können einige ihrer normalen Funktionen erfüllen. Die Anzahl von Blasten steigt auch langsamer an als bei der akuten Leukämie. Bei der chronischen Leukämie wird die Erkrankung graduell schlimmer.

Leukämie erscheint im Wesentlichen in zwei Haupttypen weißer Blutzellen, und zwar in lymphoiden Zellen oder myeloiden Zellen. Demgemäss tritt die Leukämie als lymphatische Leukämie oder myeloische Leukämie auf.

Die am häufigsten vorkommenden Leukämien sind:
_ Die Akute Lymphatische Leukämie (ALL) ist die häufigste Leukämie bei kleinen Kindern. Diese Erkrankung kann auch bei Erwachsenen, insbesondere ab dem 65. Lebensjahr, auftreten.
_ Akute Myeloische Leukämie (AML) kommt bei Erwachsenen und Kindern vor. Dieser Leukämietyp wird manchmal Akute Nicht-Lymphatische Leukämie (ANLL) genannt.
_ Chronische Lymphatische Leukämie (CLL) trifft meistens Erwachsene ab dem 55. Lebensjahr. Sie tritt manchmal auch bei jüngeren Erwachsenen auf, betrifft aber so gut wie nie Kinder.
_ Die Chronische Myeloische Leukämie (CML) kommt meist bei Erwachsenen vor. Eine geringe Zahl von Kindern entwickelt diesen Krebs auch.

### Symptome für Leukämie

Leukämische Zellen sind abnormale Zellen, welche die Funktionen normaler Blutzellen nicht ausüben können. Sie können dem Körper nicht helfen, Infektionen zu bekämpfen. Aus diesem Grund entwickeln Menschen mit Leukämie oft Infektionen und haben Fieber.

Menschen mit Leukämie weisen häufig auch weniger gesunde rote Blutzellen und Blutplättchen auf, so dass nicht genug rote Blutzellen für den Sauerstofftransport durch den Körper zur Verfügung stehen. Bedingt durch Anämie, sind die Patienten oft bleich und fühlen sich matt und müde. Ist die Anzahl der Blutplättchen zu gering, neigen die Patienten leicht zu Blutungen und entwickeln schnell blaue Hämatome.

Wie alle Blutzellen zirkulieren leukämische Zellen im Körper. Bedingt durch die Anzahl abnormaler Zellen und deren Aufenthaltsort, können Patienten mit Leukämie verschiedene Symptome zeigen.

Bei der akuten Leukämie erscheinen Symptome schnell und auch der Zustand des Patienten verschlechtert sich rasch. Bei der chronischen Leukämie erscheinen die Symptome lange Zeit nicht. Wenn Symptome erscheinen, sind sie normalerweise zuerst mild und der Allgemeinzustand des Patienten verschlechtert sich erst allmählich.

Symptome für Leukämie:
* Fieber, Frösteln und andere grippeartige Symptome;
* Schwäche und Ermüdung;
* häufige Infektionen;
* Verlust des Appetites und/oder Gewichtes;
* geschwollene oder empfindliche Lymphknoten, Leber, oder Milz;
* schnelles Bluten oder blaue Flecken;
* kleine rote Flecken unter der Haut;
* geschwollenes oder blutendes Zahnfleisch;
* Schwitzen, besonders nachts; und/oder
* Knochen- oder Gelenkschmerzen.

Bei der akuten Leukämie können sich die abnormalen Zellen im Gehirn oder im Rückenmark (zentrales Nervensystem oder CNS) sammeln. Das Resultat können dann Kopfschmerzen, Übelkeit, Brechreiz, Konfusion, Verlust der Kontrolle über die Muskeln und Schlaganfälle sein. Leukämische Zellen können sich auch in den Hoden sammeln und verursachen dort eine Schwellung. Einige Patienten entwickeln schmerzende Augen oder Haut. Leukämie kann auch den Verdauungstrakt, die Nieren, Lunge oder andere Teile des Körpers beeinflussen.

Bei der chronischen Leukämie können sich die abnormalen Zellen graduell in verschiedenen Teilen des Körpers sammeln. Chronische Leukämie kann die Haut, das CNS, den Verdauungstrakt, die Nieren und die Hoden beeinflussen.

### Behandlung von Leukämie:

Die Behandlung von Leukämie ist komplex. Sie variiert mit der Art der Leukämie und ist nicht bei allen Patienten gleich. Die Behandlung hängt auch von bestimmten Eigenarten der leukämischen Zellen, dem Ausmaß der Erkrankung, ob die Leukämie schon mal behandelt wurde, ab. Auch das Alter des Patienten, die Symptome und der generelle Gesundheitszustand sind relevante Faktoren.

Die akute Leukämie muss sofort behandelt werden. Patienten mit chronischer Leukämie, die keine Symptome zeigen, müssen nicht sofort behandelt werden. Leider kann die chronische Leukämie selten geheilt werden.

### Behandlungsmethoden:

Die meisten Patienten mit Leukämie werden chemotherapeutisch behandelt. Einige werden zusätzlich bestrahlt und/oder erhalten eine Knochenmarkstransplantation (KMT, englisch: bone marrow transplantation = BMT) oder biologische Therapien. In einigen Fällen kann eine Entfernung der Milz helfen. Vor einer Knochenmarkstransplantation erhalten die Patienten eine Ganzkörperbestrahlung in Verbindung mit einer Chemotherapie, um das Leukämie-produzierende Knochenmark zu zerstören.

Das gesunde Knochenmark kann von einem Spender kommen oder es kann vom Patienten stammen. Dann wird es vor der Hochdosisbehandlung entnommen und außerhalb des Körpers behandelt, um leukämische Zellen zu entfernen. Danach ist ein mehrwöchiger Krankenhausaufenthalt nötig, damit das Transplantat anwachsen und genügend weiße Blutkörperchen (Leukozyten), wie T-, B- Lymhpozyten, neutrophile, basophile und eosinophile Granulozyten, Thrombozyten (Blutplättchen), Monozyten u.a. Subpopulationen zur Rekonstitution (Wiederherstellung) des hämatopoetischen (blutbildenden Systems) produzieren kann. In der Zwischenzeit müssen die Patienten vor Infektionen geschützt werden.

Die biologischen Therapien beinhalten Behandlungen mit Substanzen, die die Immunantwort gegen den Krebs beeinflussen. Zytokine, wie vor allem Interferone, Interleukine und Kolonie-stimulierende Faktoren (CSF) werden z.B. bei einigen Leukämietypen verwendet. Sie werden meist mit Chemotherapie oder Knochenmarkstransplantation kombiniert.

### Knochenmarkstransplantation

Patienten mit Knochenmarkstransplantat haben ein erhöhtes Risiko für Infektionen, Blutungen und andere Nebeneffekte durch hohe Dosen der Chemotherapeutika und der Bestrahlung, die sie erhalten, bedingt. Zusätzlich kann es zu Transplantat-gegen-Empfänger-Antworten (graft versus host disease (GVHD)) kommen. Die Leber, die Haut und der Verdauungstrakt sind dabei die am häufigsten betroffenen Organe. GVHD kann mild oder schwerwiegend sein und jederzeit nach der Transplantation auftreten (auch Jahre später). Immunsuppressiva werden verabreicht, um das Risiko des GVHD zu vermindern und zu behandeln.

### Chronische Myeloide Leukämie

Am Beispiel der Chronischen Myeloiden Leukämie wird im folgenden die Wirkungsweise der Knochenmarkstransplantate erläutert:
Allologe Knochenmarkstransplantationen (bone-marrow transplantation (BMT)) stellten in den vergangenen 20 - 30 Jahren Behandlungsmöglichkeiten für die chronische myeloide Leukämie (CML) bereit. Allerdings lassen sich für etwa 60% der Patienten keine geeigneten Spender finden.

CML ist in der frühen chronischen Phase durch eine einzelne ermittelte transformierende genetische Abnormalität charakterisiert. Es handelt sich dabei um die t(9;22) Translokation (Philadelphia Chromosome, Ph), die das bcr-abl Oncogen kreiert, den einzigen Faktor, der unbedingt für die Entwicklung der Krankheit benötigt wird (Daley et al 1990). Verglichen mit anderen Tumortypen in der chronischen Phase besitzen CML Patienten ein relativ intaktes Immunsystem (Lewalle et al 1996). CML ist für eine tumorspezifische Immunantwort zugänglich. In den vergangenen 20 - 30 Jahren wurden Anti-Tumor-Antworten von allologen Knochenmarkstransplantaten (allo-BMT) und zuletzt auch von Spender-Leukozyten-Infusionen [Donor leukocyte infusion (DLI)] klinisch zur Behandlung von CML ausgenutzt.
Die Erkennung und Auslöschung von verbleibenden Tumorzellen durch Spenderimmunzellen scheint essentiell für die Induktion einer molekularen Remission zu sein. Transplantate, aus denen die T-Zellen entfernt wurden, vergrößern das Risiko des Rückfalls zur CML (Champlin et al 1988; Horowitz et al 1990). Eine stringente Demonstration, des durch allo-BMT generierten Anti-Leukämie-Effektes kann man beobachten, wenn DLI verabreicht wird, sobald die Krankheit wiedererscheint. In dieser Konstellation kann DLI eine anhaltende molekulare Remission in bis zu 70% der Fälle wiedereinsetzen (MacKinnon 2000). DLI steht aber auch mit einer signifikanten Toxizität, verursacht durch eine GVHD in Verbindung. Diese begleitet oft den Transplantat-gegen-Leukämie-(GVL)-Effekt. Die Mortalität liegt hierbei bei 50-90% (MacKinnon 2000).

Hinweise für Immunregulationen bei der CML:
_ Erhöhtes Risiko eines Rückfalls bei T-Zell-Exklusion aus dem Transplantat
_ Erhöhtes Risiko eines Rückfalls bei Abwesenheit von GVHD
_ BMT von syngenen Zwillingen ist weniger effektiv als passendes BMT von Geschwistern
_ Der Rückfall reagiert auf eine Absetzung einer Immunsuppression
_ Spender-Leukozyten-Infusionen sind bei einem Rückfall effektiv

Die Allo-BMT stellt einen ziemlich groben Ansatz mit signifikanter Transplantatbedingter Morbidität und Mortalität dar. Das Risiko zu sterben liegt bei 20 - 41% (Silver et al 1999). Diese Form der Immunotherapie bietet aber auch eine bis zu 70% Leukämie-freie Überlebensrate für die Transplantatempfänger (Clift & Anasetti 1997). Diese GvHD überlagert den gewünschten antileukaemischen Effekt.

Knochenmarkstransplantationen werden auch bei anderen Leukämien angewandt.

### Graft-versus-Host Disease (GvHD)

Die "graft versus host disease" (GvHD) ist eine Hauptursache von Morbidität und Mortalität nach allogener Transplantation und wird durch Spender-T-Lymphozyten im Transplantat verursacht. Bei HLA-kompatiblen Transplantationen ist die GvHD-Antwort gegen Nebenhistokompatibilitätsantigene (minor histocompatibility antigens) gerichtet. Vermutlich präsentieren-APC des Empfängers die fremden Gewebsantigene den Spender-T-Lymphozyten, insbesondere, da die Aktivität der APC durch Chemo- und radiotherapieinduzierte Zytokine wie Interleukin (IL) -1, IL-6 und TNFa erhöht ist.
Die GvHD betrifft vor allem Haut (kleinfleckiges Exanthem), Leber (cholestatische Hepatitis) und Darm (Diarrhoe). Ferner kommt es zur Austrocknung von Schleimhäuten und Bindehaut (Sicca-Syndrom) und zur Myositis. Zur Prophylaxe einer GvHD führt man eine Immunspuppression mit Cyclosporin durch, evtl. in Kombination mit Methotrexat und Kortikosteroiden.

### T-Zell-Depletion und Graft-versus-leukemia-Effekt

Durch Depletion von T-Lymphozyten im Transplantat sinkt das Risiko einer GvHD. Gleichzeitig steigt allerdings das Risiko eines Tumorrezidivs, da die Spender-T-Lymphozyten auch den günstigen Graft-versus-leukemia-Effekt (GvL) vermitteln. Will man den GvL-Effekt bei gleichzeitiger Reduzierung des GvHD-Risikos beibehalten, muss man die T-Zellen partiell depletieren und die Spender-T-Lymphozyten zu späteren Zeitpunkten schrittweise reinfundieren.

### Fas und Trail

FasL ist als ein potentes Apoptose-induzierendes Molekül (Membranprotein) bekannt. Es vermittelt seine Wirkung über Fas einen Rezeptor, der auf T-Zellen vorkommt. Werden DCs mit FasL transfiziert, so induzieren sie Apoptose in T-Zellen (Matsue et al 1999). FasL-transfizierte DCs können eine Agspezifische Verminderung einer Transplantatabstossung bewirken. Dies beruht auf der Wechselwirkung (Apoposeinduktion) von FasL auf DCs und Fas-Rezeptor auf den T-Zellen (Min et al 2000).

TRAIL (tumor necrosis factor-related apoptosis-inducing ligand) ist ein kürzlich identifiziertes Mitglied der Tumor-Necrosis-Faktor-Cytokine Superfamilie. TRAIL induziert in verschiedenen Tumorzellinien Apoptose. Es wird auch vermutet, daß TRAIL beim Tod von Lymphozyten eine Rolle spielt (Simon et al 2001; Miura et al 2001).

### Co-stimulatorische Moleküle

Funktionelle, reife DCs entstehen aus zirkulierenden Vorläuferzellen nach einer Reifungsperiode. DCs können durch einen spezifischen Besatz Markermolekülen charakterisiert werden. Zu diesen gehören akzessorische, kostimulatorische Genprodukte wie CD40, CD80, und CD86 sowie MHC Klasse I und II. Besonders das CD83 Molekül ist ein gut charakterisierter Marker für vollständig ausgereifte DCs, da CD83 nicht auf unreifen DC-Vorläufern detektiert werden kann. Die funktionelle Signifikanz von CD83 ist aber unbekannt. Seine stärker werdende Präsenz im Verlauf der Reifung deutet auf eine wichtige Funktion hin (Berchtold et al 1999).
Herpes Simplex Virus vermag es auf nicht geklärte Weise, die Expression von CD83 zu unterdrücken, während die Produktion anderer für DCs typischer Moleküle wie CD25, CD40, CD80, CD86, CD95 und MHC der Klasse I und der Klasse II davon unbeeinflusst bleibt (Kruse et al 2000b). Interessanterweise führt die Inhibition der CD83 Expression an der Plasmamembran zu einer dramatischen Reduktion der DC-vermittelten T-Zell-Stimulation. Wird durch einen spezifischen Inhibitor der eukaryotische Initiationsfaktor 5A behindert, kann die CD83 Expression verhindert werden, was auch zu einer starken Reduktion der DC-vermittelten T-Zell-Stimulation führt (Kruse et al 2000a). eIF-5A ist ein Protein, welches in den Exportpfad von spezifischen RNAs aus dem Zellkern einbezogen ist.
eIF-5A ist das einzige zelluläre Protein von dem man weiß, dass es die ungewöhnliche Aminosäure Hypusin enthält. Diese Modifikation scheint für die Zellteilung nötig zu sein. Die Hypusinmodifikation ist eine Spermidin-abhängige posttranslationale Reaktion, die von zwei Enzymen katalysiert wird. Das beinhaltet den Transfer der Aminobutylgruppe des Spermidin auf die ε-NH₂-Gruppe von Lysin an Position 50 in eIF-5A durch die Deoxyhypusinsynthase (Kruse et al 2000a).
Das Intermediat, welches dabei generiert wird, wird anschließend durch die Deoxyhypusinhydroxylase hydroxyliert. Auf diese Art entsteht die aktive Form von eIF-5A. Obwohl eIF-5A ursprünglich als "Initiationsfaktor" bezeichnet wurde, zeigten jüngere *in vitro* und *in vivo* Experimente, daß eIF-5A kein Initiator der Proteintranslation ist. eIF-5A scheint ein Teil eines spezifischen Exportpfades aus dem Zellkern zu sein, welcher z.B. von der Rev/Rex Klasse der retroviralen RNA Transportfaktoren ausgenutzt wird. Außerdem wurde gezeigt, dass das eIF-5A Protein sich an der nucleoplasmischen Seite des Kernporenkomplexes sammelt, um mit dem allgemeinen Kernexportrezeptor CRM1 zu interagieren und um in Säugerzellen vom Nucleus in das Zytoplasma zu wandern. Untersuchungen der eIF-5A mRNA-Menge in humanen Zellen zeigte, dass eIF-5A in Zellinien, sowie verschiedenen anderen Geweben konstitutiv exprimiert wird. Im Gegensatz dazu wird das eIF-5A Gen in primären lymphoiden Zellen während der Aktivierung und/oder Proliferation von primären human Blutzellen induziert. Benutzt man einen Inhibitor der Hypusinmodifikation [GC7 (N¹ -guanyl-1,7-diaminoheptan)], so wird die Expression von CD83 und damit die volle stimulatorische Aktivität reifer DCs inhibiert (Kruse et al 2000a).

Vorzugsweise ist die erfindungsgemäße Zelle eine dendritische Zelle oder eine andere Antigen-präsentierende Zelle oder einer der Vorläufer einer solchen Zelle.

Die APCs sind die Schaltstellen des anpassungsfähigen Immunsystems. Nur sie können eine T-Zell-vermittelte Immunantwort auslösen oder abstellen.

Die allologen Immunreaktionen - lassen sich nicht aufrechterhalten ohne die Hilfe und Vermittlung von APCs und T-Helfer-Lymphozyten. Auch die cytotoxischen T-Zellen werden von APCs aktiviert. Sie vermitteln eine zelluläre Immunantwort, bei der Zellen direkt von den cytotoxischen T-Zellen getötet werden.

Die molekularen Mechanismen der Interaktion - des Zell-Zell-Kontakts - von APCs mit den T-Helfer- und cytotoxischen T-Lymphozyten ist auf Rezeptorebene bis ins Detail bekannt.

Handelt es sich bei dem Antigen z.B. um ein Bakterienprotein, ist die Immunreaktion für den Organismus nützlich. Ist das Antigen allerdings eine Transplantatantigen, spricht man von einer (pathologischen) Abstoßungsreaktion.

Neben dem zu präsentierenden Antigen (gegen welches sich die zu produzierenden Antikörper richten) sind auf der Zelloberfläche verschiedene Rezeptoren und Hilfsrezeptoren am Zell-Zell-Kontakt und der Zellaktivierung beteiligt. Ein essentielles Molekül der interzellulären Wechselwirkung bei der Antigenpräsentation (Kontakt von APCs mit den T-Lymphozyten) sind costimulierende Rezeptoren mit der Bezeichnung B7 (CD80, CD86).

WO 0129192 offenbart eine Methode, Antigen-präsentierende Zellen zur Verhinderung von GvHD-Effekten zu verwenden. Die Antigen-präsentierenden Zellen werden hierbei allerdings mit toten oder sterbenden Zellteilen des Transplantatempfängers koinkubiert, um beladene Antigen-präsentierende Zellen zu erhalten. Die Zellbestandteile sollen dabei Antigene enthalten, für welche eine Reduktion einer Immunantwort gewünscht ist. Dieser Schritt wird erfindungsgemäß nicht benötigt, da Zellen des Empfängers benutzt werden. Außerdem werden weitere Veränderungen herbeigeführt oder unreife DCs verwandt, um eine abstoßende Immunantwort zu vermindern.

Das der Erfindung zu Grunde liegende Problem besteht unter anderem darin, therapeutisch nutzbare Produkte für die Reduktion von allologen Immunreaktionen bei Knochenmarkstransplantationen zur Verfügung zustellen.
Gelöst wird das Problem durch Zellen des Empfängers, die Antigen-präsentierende Zellen sind oder Zellen sind, aus denen Antigen-präsentierende Zellen generiert werden können. Bei den Antigen-präsentierenden Zellen sind insbesondere die dendritischen Zellen, aber auch B-Zellen von Interesse. Die Antigen-präsentierenden Zellen sollen mit allologen Knochenmarkstransplantaten inkubiert werden. Diese Transplantate enthalten Lymphozyten. Die Antigen-präsentierenden Zellen können in einer bevorzugten Ausführungsform auch allein mit den Lymphozyten oder T-Zellen (T-Lymphozyten) inkubiert werden. Die Antigen-präsentierenden Zellen können in einer bevorzugten Ausführungsform auch allein mit den Lymphozyten oder T-Zellen (T-Lymphozyten) inkubiert werden. Unter den Lymphozyten sind die T-Zellen von besonderem Interesse, da diese für den Empfänger/Patienten wichtige Reaktionen auslösen. Eine der Reaktionen ist gewollt und nützlich, die andere ist schädlich und führt oft zum Ableben des Empfängers. Bei der nützlich Reaktion handelt es sich um die antileukämische GvL, bei der schädlichen um die GVHD. Die Inkubation der Antigen-präsentierenden Zellen mit dem Transplantat und hier besonders mit den darin vorkommenden T-Zellen soll die GvHD-Reaktion abschalten und nur die GvL-Reaktion erhalten. Zu diesem Zweck werden die Antigen-präsentierenden Zellen so gewählt oder behandelt, dass sie T-Zellen nicht mehr aktivieren können, sehr wohl aber noch die Alloantigene des Empfängers präsentieren. Trifft eine alloreaktive T-Zelle auf eine solche Antigen-präsentierende Zelle, so wird sie dauerhaft stillgestellt, oder in den programmierten Zelltod (Apoptose) geschickt, oder zur regulatorischen T-Zelle umfunktioniert. Als regulatorische T-Zelle wird eine T-Zelle verstanden, die Immunantworten gegen spezifische Antigene, hier Alloantigene verhindert. Im folgenden sind Möglichkeiten aufgezeigt, dass Problem zu lösen:

### A.

Gelöst wird das Problem durch die erfindungsgemäße Zelle, die vom Empfänger des Transplantats stammt und dadurch gekennzeichnet ist, dass eine der Funktionen co-stimulatorischer Rezeptoren, wie ein CD83-Rezeptor und/oder B7- und/oder CD40-Rezeptor. supprimiert ist und/oder CTLA4-bindende Moleküle hinzugegeben werden, die CTLA4 an T-Zellen des Transplantats binden und/oder Fas-bindende Moleküle hinzugegeben werden, die Fas an T-Zellen des Transplantats binden und/oder TRAIL-Rezeptorbindende Moleküle hinzugegeben werden, die TRAIL-Rezeptoren an T-Zellen des Transplantats binden, oder Interleukin 10 (IL-10) hinzugegeben wird. Die Moleküle, die CD83, CD80, CD86, CD40 binden, blockieren costimulatorische Rezeptoren auf APCs. Durch die Blockade der Costimulation werden T-Zellen abgeschaltet und/oder sie werden in den programmierten Zelltod (Apoptose) getrieben und/oder sie differenzieren zu regulatorischen T-Zellen, wenn die T-Zellen gleichzeitig ein präsentiertes Antigen mit ihrem T-Zell-Rezeptor erkennen. Durch Moleküle, die Fas und/oder TRAIL-Rezeptoren an T-Zellen des Transplantats binden, können T-Zellen in den programmierten Zelltod (Apoptose) getrieben werden, wenn diese gleichzeitig ein präsentiertes Antigen mit ihrem T-Zell-Rezeptor erkennen. IL-10 behindert die Aktivierung von T-Zellen (Corinti et al 2001). Bei den bei dieser Methode erkannten Antigenen handelt es sich vorwiegend um Transplantationsantigene (Haupthistokompatibilitätskomplexe (HLA, MHC I, MHC II), Rhesus Faktor, Nebenhistokompatibilitätsantigene (minor histocompatibility antigens)), da diese sich zwischen Spender und Empfänger des Transplantates unterscheiden. In einer bevorzugten Ausführung werden die Bindemoleküle vernetzt, was z.B. durch andere Bindemoleküle, wie z.B. Antikörper, geschehen kann, die wiederum die Bindemoleküle gegen CD83, CD80, CD86, CD40, CTLA4, Fas, TRAIL-Rezeptoren binden. Ein Vernetzen befördert den gewünschten Effekt.

### B.

Die erfindungsgemäßen Zellen können auch ganz oder teilweise durch unreife dendritische Zellen (DC; z.B. *in vitro* abgeleitete) dargestellt werden. Durch die bei unreifen DCs fehlende Costimulation werden T-Zellen (wie in A.) abgeschaltet und/oder sie werden in den programmierten Zelltod (Apoptose) getrieben und/oder sie differenzieren zu regulatorischen T-Zellen. Die unreifen DCs verhalten sich wie DCs, deren Costimulation (siehe A.) geblockt wird (Dhodapkar et al 2001).

### C.

Die erfindungsgemäße Zelle kann auch mit mindestens einem Gen für ein Fas-Bindemolekül transfiziert sein. Durch die Expression von FasL an der Plasmamembran von dendritischen Zellen können T-Zellen (wie in A.) zum programmierten Zelltod (Apoptose) gezwungen werden.

### D.

Die erfindungsgemäße Zelle kann auch mit mindestens einem Gen für ein Tumor-Nekrosis-Faktor-related apoptosis-inducing ligand (TRAIL)-Rezeptor-Bindemolekül transfiziert sein. Durch die Expression von TRAIL-Rezeptor-Bindemolekülen an der Plasmamembran von dendritischen Zellen können T-Zellen (wie in A.) zum programmierten Zelltod (Apoptose) gezwungen werden.

### E.

Die erfindungsgemäße Zelle kann auch durch Zugabe von Aspirin, 1a,25 Dihydroxyvitamin D3, 1a,25(OH)2-16-ene-23-yne-26,27-hexafluoro-19-norvitamin D3 (D3 analog), Vitamin D3, Glucocorticoide behandelt werden (Adorini et al 2001; Griffin et al 2001; Griffin et al 2000; Hackstein et al 2001; Penna & Adorini 2000; Penna & Adorini 2001)(WO0124818). Durch diesen Eingriff verbleiben dendritische Zellen in einem Zustand, der die immunstimmulatorischen Eigenschaften der dendritischen Zellen einschränkt. Durch die reduzierte Costimulation werden T-Zellen (wie in A.) abgeschaltet und/oder sie werden in den programmierten Zelltod (Apoptose) getrieben und/oder sie differenzieren zu regulatorischen T-Zellen.

### F.

Die erfindungsgemäße Zelle kann auch mit mindestens einem Gen für ein CTLA4-Bindemoleküle transfiziert sein. Durch die Expression von CTLA4-Bindemolekülen an der Plasmamembran von dendritischen Zellen können T-Zellen abgeschaltet und/oder in den programmierten Zelltod (Apoptose) getrieben und/oder zu regulatorischen T-Zellen differenziert werden.

### G.

Die erfindungsgemäße Zelle kann auch mit mindestens einem Gen für ein CD83-Bindemolekül und/oder für B7 (CD80/CD86)-Bindemoleküle und/oder für ein CD40-Bindemolekül transfiziert sein. Durch die Expression von CD83-Bindemolekülen und/oder B7 (CD80/CD86)-Bindemolekülen und/oder CD40-Bindemolekülen im endoplasmatischen Retikulum und/oder Golgi und/oder extrazellulär und/oder an der Plasmamembran von dendritischen Zellen können T-Zellen abgeschaltet und/oder in den programmierten Zelltod (Apoptose) getrieben und/oder zu regulatorischen T-Zellen differenziert werden.

### H.

Die erfindungsgemäße Zelle kann auch mit mindestens einem Gen für IL-10 transfiziert sein. Durch die Expression von IL-10 von dendritischen Zellen werden die dendritischen Zellen an der Aktivierung von T-Zellen gehindert (Corinti et al 2001), sodass T-Zellen abgeschaltet werden können und/oder in den programmierten Zelltod (Apoptose) getrieben und/oder zu regulatorischen T-Zellen differenziert werden.

### I.

Die erfindungsgemäße Zelle kann durch Inkubation mit den Interferonen (IFNa oder IFNy oder IFNβ) zur Synthese von Tumor-Nekrosis-Faktor-related apoptosis-inducing ligand (TRAIL) veranlaßt werden. Durch die Expression von TRAIL- an der Plasmamembran von dendritischen Zellen können T-Zellen (wie in A.) zum programmierten Zelltod (Apoptose) gezwungen werden (Liu et al 2001; Fanger et al 1999).

Eine der Prozeduren (A.-I.) und/oder eine beliebige Abfolge dieser Prozeduren (A.-I.) wird angewandt, um die erfindungsgemäße Zelle *in vitro* mit allogenen Stammzellen (z.B. inklusive von Lymphozyten) oder mit allogenen T-Zellen oder mit allogenen Lymphozyten eines fremden Stammzell-Transplantatspenders (z.B. Knochenmark) zu inkubieren. Die genannten Zellen, die aus dem Empfänger des Transplantates stammen und bei Krebskranken, insbesondere solchen mit Leukämien, Krebszellen enthalten können, können in einer bevorzugten Ausführungsform des erfindungsgemäßen Verfahrens vor der Inkubation mit dem Transplantat so behandelt werden, dass eine Proliferation dieser Zellen ausbleiben wird. Vorzugsweise wird dies durch Bestrahlung dieser Zellen erreicht.

Die durch eine dieser Prozeduren und/oder eine beliebige Abfolge dieser Prozeduren positiv selektierten nicht alloreaktiven, aber allologen T Zellen werden dem Patienten zusammen mit den allologen Stammzellen (z.B. Knochenmark) infundiert, vorzugsweise ins Knochenmark. Auf diese Weise erhält man einen Graft-versus-Leukämie-Effekt (GvL), aber keinen, oder aber einen wesentlich verringerten Graft-versus-Host-Disease (GvHD).

Die CD83 bindenden Moleküle können vorzugsweise Antikörper, monoklonale Antikörper, F(ab)_{2,} Einzelkettenantikörper (scFv), und/oder F_{ab}-Fragmente sein.
Die Proteine, die affine Strukturen zu B7-Rezeptoren aufweisen, können vorzugsweise CTLA4, CTLA4-Abkömmlinge (z.B. CTLA4Ig), CD28, Antikörper, F(ab)₂, scFv und/oder F_{ab}-Fragmente sein.
Die Proteine, die affine Strukturen zu CD40-Rezeptoren aufweisen, können vorzugsweise CD40L, CD40L-Abkömmlinge, Antikörper, F(ab)₂, scFv und/oder F_{ab}-Fragmente sein.
Die Proteine, die affine Strukturen zu CTLA4 (CD152)-Rezeptoren aufweisen, können vorzugsweise Antikörper, F(ab)₂, scFv und/oder F_{ab}-Fragmente sein.
Die Proteine, die affine Strukturen zu Fas-Liganden aufweisen, können vorzugsweise FasL, FasL-Abkömmlinge, Antikörper, F(ab)₂, scFv und/oder F_{ab}-Fragmente sein.
Die Proteine, die affine Strukturen zu TRAIL-Rezeptoren aufweisen, können vorzugsweise TRAIL, TRAIL-Abkömmlinge, Antikörper, F(ab)_{2,} scFv und/oder F_{ab}-Fragmente sein.

Das erfindungsgemäße Verfahren kann auf gentechnologischen Eingriffen an patienteneigenen Zellen (Prä-Zellen: Stammzellen, Monozyten, DCs, CD34+ Stammzellen, CD34+ Progenitor-Stammzellen und/oder B-Zellen) beruhen. Die Eingriffe erfolgen mittels geeigneter Sonden und bewirken die Verminderung von CD83-Molekülen und gegebenenfalls B7-Molekülen und/oder CD40-Molekülen durch die Behinderung bzw. Verhinderung der Ausbildung des Moleküls auf der Oberfläche der Antigen-präsentierende Zellen und/oder CTLA4 bindenden Molekülen, vorzugsweise Antikörper, und/oder die Produktion von Fas bindenden Molekülen vorzugsweise FasL und/oder die Produktion von TRAIL-Rezeptor bindenden Molekülen vorzugsweise TRAIL und/oder die Produktion von IL-10.
Eine Transfektion der Prä-zellen (Stammzellen, Monozyten, DCs, CD34+ Stammzellen, CD34+ Progenitor-Stammzellen und/oder B-Zellen) oder Antigen-präsentierenden Zellen bewirkt eine Expression von Fas-Bindemolekülen und/oder TRAIL-Rezeptor-Bindemolekülen und/oder IL-10 und/oder B7-Bindemolekülen und/oder CTLA4-Bindemolekülen und/oder CD83-Bindemolekülen und/oder CD40-Bindemolekülen bewirkt.
Dies wird durch Nukleinsäuren, die für Fas-Bindemoleküle und/oder TRAIL-Rezeptor-Bindemoleküle und/oder IL-10 und/oder B7-Bindemoleküle und/oder CTLA4-Bindemoleküle und/oder CD83-Bindemoleküle und/oder CD40-Bindemoleküle kodieren, erreicht.
Bei den Nukleinsäuren kann es sich um DNA, RNA, Oligonukleotide, Polynukleotide, Ribozyme oder Peptidnukleinsäuren (PNA) handeln.
In einer bevorzugten Ausführungsform enthält die DNA Regulationselemente wie Enhancer, Promotoren, polyA-kodierende 3'-Enden zur Transkription der DNA in RNA. Die RNA wiederum sollte Regulationselemente zur Translation der RNA in Protein enthalten.
Bei einigen der beschriebenen Expressionsprodukte (Fas-, TRAIL-Rezeptorund CTLA4-Bindemoleküle) wird eine Lokalisation in der Plasmamembran der Prä-zellen und/oder APCs angestrebt. Dafür werden die Nukleinsäuren mit einer Signalsequenz versehen, die den Transport der Expressionsprodukte zur Plasmamembran bewirkt.
Bei anderen beschriebenen Expressionsprodukten (B7-, CD83- und CD40-Bindemoleküle) wird in einer bevorzugten Ausführungsform eine Lokalisation im endoplasmatischen Retikulum, dem Golgi-Apparat, dem Trans-Golgi-Netzwerk oder intrazellulären Vesikeln angestrebt, Zu diesem Zweck kodieren die Nukleinsäuren eine Signalsequenz die den Verbleib der Expressionsprodukte in den jeweiligen Zellkompartimenten bewirkt.

Die genannten Zellen können ex vivo durch Behandlung mit Viren, viralen Vektoren, bakteriellen Vektoren, Plasmiden, die durch viralen Gentransfer, Elektroporationstechniken, Iontophorese, ballistischen Methoden und/oder anderen Techniken zur Einschleusung von Molekülen in eukaryotische Zellen geeignet sind, transfiziert werden.

Eine genannte Zelle kann durch Behandlung mit Viren, viralen Vektoren, bakteriellen Vektoren, Plasmiden die durch viralen Gentransfer, Elektroporationstechniken, Iontophorese, ballistischen Methoden und/oder anderen Techniken zur Einschleusung von Molekülen in eine Zelle mit erhöhter Produktion von Fas-Bindemolekül und/oder TRAIL-Rezeptor-Bindemolekül und/oder CD83-Bindemoleküle, CD40-Bindemoleküle, B7 (CD80/CD86)-Bindemoleküle, CTLA4-Bindemolekül, IL-10 transfiziert werden, wodurch T-Zellen, welche über allologe Antigene, die z.B. an MHC-Molekülen präsentiert werden, an die Antigen-präsentierende Zelle binden, ausgeschaltet werden.

Als Bindemoleküle können Moleküle wie Antikörper, Proteine, Peptide, Peptidomimetica, CTLA4, CTLA4-Derivate wie CTLA4Ig, CD28, CD40L und/oder Bestandteile und/oder Kombinationen dieser Moleküle, die z.B. B7-1, B7-2, CD83, CD40 binden, dienen. Durch diese Moleküle wird eine in Gegenwart einer Alloantigenpräsentation stattfindende Stimulation und/oder Co-Stimulation von T-Zellen behindert, die mit der erfindungsgemäßen Zelle in Kontakt gebracht werden.

Die Moleküle (Fas-Bindemolekül, TRAIL-Rezeptor-Bindemolekül, CD83-Bindemoleküle, CD40-Bindemoleküle, B7 (CD80/CD86)-Bindemoleküle, CTLA4-Bindemolekül, IL-10-Gen) können durch Vehikel, wie Liposomen, Hydrogele, Zyklodextrine, Nanokapseln, Nanopartikel, bio-adhäsive Mikrokugeln und/oder durch Elektroporationstechniken, Iontophorese, ballistische Methoden und/oder andere Techniken zur Einschleusung von Molekülen in die erfindungsgemäße Zelle transferiert werden.

Nukleinsäuren können insbesondere durch Viren, virale Vektoren, bakterielle Vektoren, Plasmide, die durch Elektroporationstechniken, Iontophorese, ballistische Methoden und/oder andere Techniken zur Einschleusung von Molekülen in die monoantigene Antigen-präsentierende Zelle und/oder die Antigen-präsentierende Zelle transferiert werden.

Die APC oder Prä-zelle die aus der Gruppe bestehend aus Stammzellen, Monozyten, DCs, CD34+ Stammzellen, CD34+ Progenitor-Stammzellen und/oder B-Zellen ausgewählt wird, kann mit einem Inhibitor der Hypusinbiosynthese, wie z.B. GC7 (N¹ -guanyl-1,7-diaminoheptane) inkubiert werden, um die Produktion von funktionalem eIF-5A zu behindern, was wiederum die CD83-Biosynthese behindert.

Erfindungsgemäß beansprucht wird weiterhin ein Arzneimittel, enthaltend mindestens eine erfindungsgemäße Zelle. Vorzugsweise ist das erfindungsgemäße Arzneimittel als Infusionslösung zur intravenösen oder intraperitonealen Applikation formuliert. Die Formulierung ist so gewählt, dass bei Verabreichung des Arzneimittels keine wesentliche Beeinträchtigung der Wirksamkeit der erfindungsgemäßen Antigen-präsentierenden Zelle erfolgt.

So kommt als Infusionslösung vorzugsweise physiologische Kochsalzlösung in Betracht. Grundsätzlich sind auch andere Lösungen, die einen pH-Wert von 5,5 bis 8,5 aufweisen, geeignet. Auch Serum, beispielsweise humanes Serum, autologes Serum oder Serum anderer Spezies, Lösungen mit Plasmaersatzstoffen, wie Polyvinylpyrrolidon, kommen in Betracht. Typischerweise sollen 0,5 ml bis 500 ml appliziert werden. Diese Mengen und pH-Werte sind selbstverständlich nicht absolut, sondern können vom Fachmann, je nach Bedingungen und Anforderungen, variiert und an die spezifischen Bedürfnisse eines Patienten angepasst werden.

Die erfindungsgemäße Antigen-präsentierende Zelle kann insbesondere zur Herstellung eines Arzneimittels zur Behandlung von Immunreaktionen gegen allologe Gewebsmerkmale bzw. zur Prävention von GvHD verwendet werden.

Es wird erfindungsgemäß eine Verwendung beansprucht, bei der die zu behandelnden Immunreaktionen in Verbindung mit allologen Gewebsmerkmalen, deren Gensequenzen und/oder Teilsequenzen, insbesondere Haupthistokompatibilitätscomplexe, MHC I, MHC II, Rhesus Faktor, Nebenhistokompatibilitätsantigene (minor histocompatibility antigens) stehen.

Es wird erfindungsgemäß eine Verwendung beansprucht, bei der die Bindemoleküle in einer bevorzugten Ausführung vernetzt werden, was durch andere Bindemoleküle, wie z.B. Antikörper, geschehen kann, die wiederum die Bindemoleküle gegen CD83, CD80, CD86, CD40, CTLA4, Fas, TRAIL-Rezeptoren binden. Eine solche Vernetzung verstärkt den Effekt dieser Bindemoleküle.

Es wird auch erfindungsgemäß eine Verwendung beansprucht, bei der die *ex vivo* selektierten Zellen dem Patienten infundiert werden und dieser danach einer *in vivo* Behandlung mit IL-2 unterliegt. Die *ex vivo* selektierten Zellen können auch *ex vivo* mit IL-2 oder anderen Stimulanzien, wie Phytohemaglutinin (PHA) inkubiert und danach dem Patienten infundiert werden. Es hat sich gezeigt, daß so der GvL-Effekt verstärken werden kann. Dieser Effekt ist in der Literatur beschrieben (Klingemann HG 1995; Sykes M 1994; Uharek L 1998; Vourka-Karussis U 1995; Weiss L 1995; Weiss L 1999; Xun CQ 1995).

### Literatur

Adorini, L., Penna, G., Casorati, M., Davalli, A. M., Gregori, S. 2001. Induction of transplantation tolerance by 1,25-dihydroxyvitamin D(3). *Transplant Proc* 33:58-9.
Berchtold, S., Muhl-Zurbes, P., Heufler, C., Winklehner, P., Schuler, G., Steinkasserer, A. 1999. Cloning, recombinant expression and biochemical characterization of the murine CD83 molecule which is specifically upregulated during dendritic cell maturation. *FEBS Lett* 461:211-6.
Corinti, S., Albanesi, C., la Sala, A., Pastore, S., Girolomoni, G. 2001. Regulatory activity of autocrine IL-10 on dendritic cell functions. J *Immunol* 166:4312-8.
Dhodapkar, M. V., Steinman, R. M., Krasovsky, J., Munz, C., Bhardwaj, N. 2001. Antigen-specific inhibition of effector T cell function in humans after injection of immature dendritic cells. *J Exp Med* 193:233-8.
Fanger, N. A., Maliszewski, C. R., Schooley, K., Griffith, T. S. 1999. Human dendritic cells mediate cellular apoptosis via tumor necrosis factor-related apoptosis-inducing ligand (TRAIL). *J Exp Med* 190:1155-64.
Griffin, M. D., Lutz, W., Phan, V. A., Bachman, L. A., McKean, D. J., Kumar, R. 2001. Dendritic cell modulation by lalpha,25 dihydroxyvitamin D3 and its analogs: a vitamin D receptor-dependent pathway that promotes a persistent state of immaturity in vitro and in vivo. *Proc Natl Acad Sci U S A* 98:6800-5. Griffin, M. D., Lutz, W. H., Phan, V. A., Bachman, L. A., McKean, D. J., Kumar, R. 2000. Potent inhibition of dendritic cell differentiation and maturation by vitamin D analogs. *Biochem Biophys Res Commun* 270:701-8.
Hackstein, H., Morelli, A. E., Larregina, A. T., Ganster, R. W., Papworth, G. D., et al. 2001. Aspirin inhibits in vitro maturation and in vivo immunostimulatory function of murine myeloid dendritic cells. *J Immunol* 166:7053-62.
Klingemann HG, P. G. 1995. Is there a place for immunotherapy with interleukin-2 to prevent relapse after autologous stem cell transplantation for acute leukemia? *Leuk Lymphoma* 16:397-405
Kruse, M., Rosorius, O., Kratzer, F., Bevec, D., Kuhnt, C., et al. 2000a. Inhibition of CD83 cell surface expression during dendritic cell maturation by interference with nuclear export of CD83 mRNA. *J Exp Med* 191:1581-90.
Kruse, M., Rosorius, O., Kratzer, F., Stelz, G., Kuhnt, C., et al. 2000b. Mature dendritic cells infected with herpes simplex virus type 1 exhibit inhibited T-cell stimulatory capacity. *J Virol* 74:7127-36.
Liu, S., Yu, Y., Zhang, M., Wang, W., Cao, X. 2001. The involvement of tnfalpha-related apoptosis-inducing ligand in the enhanced cytotoxicity of ifnbeta-stimulated human dendritic cells to tumor cells. *J Immunol* 166:5407-15.
Matsue, H., Matsue, K., Walters, M., Okumura, K., Yagita, H., Takashima, A. 1999. Induction of antigen-specific immunosuppression by CD95L cDNAtransfected 'killer' dendritic cells. *Nat Med* 5:930-7.
Min, W. P., Gorczynski, R., Huang, X. Y., Kushida, M., Kim, P., et al. 2000. Dendritic cells genetically engineered to express Fas ligand induce donorspecific hyporesponsiveness and prolong allograft survival. *J Immunol* 164:161-7.
Miura, Y., Misawa, N., Maeda, N., Inagaki, Y., Tanaka, Y., et al. 2001. Critical contribution of tumor necrosis factor-related apoptosis- inducing ligand (TRAIL) to apoptosis of human CD4+ T cells in HIV-1- infected hu-PBL-NOD-SCID mice. *J Exp Med* 193:651-60.
Penna, G., Adorini, L. 2000. 1 Alpha,25-dihydroxyvitamin D3 inhibits differentiation, maturation, activation, and survival of dendritic cells leading to impaired alloreactive T cell activation. *J Immunol* 164:2405-11.
Penna, G., Adorini, L. 2001. Inhibition of costimulatory pathways for T-cell activation by 1,25- dihydroxyvitamin D(3). *Transplant Proc* 33:2083-4.
Simon, A. K., Williams, O., Mongkolsapaya, J., Jin, B., Xu, X. N., et al. 2001. Tumor necrosis factor-related apoptosis-inducing ligand in T cell development: sensitivity of human thymocytes. *Proc Natl Acad Sci U S A* 98:5158-63.
Sykes M, H. M., Szot GL, Pearson DA. 1994. Interleukin-2 inhibits graft-versus-host disease-promoting activity of CD4+ cells while preserving CD4-and CD8-mediated graft-versus-leukemia effects. *Blood* 83:2560-2569.
Uharek L, G. B., Zeis M, Dreger P, Steinmann J, Loffler H, Schmitz N. 1998. Abrogation of graft-vs.-leukemia activity after depletion of CD3+ T cells in a murine model of MHC-matched peripheral blood progenitor cell transplantation (PBPCT). *Exp Hematol* 26:93-99
Vourka-Karussis U, K. D., Ackerstein A, Slavin S. 1995. Enhancement of GVL effect with rhIL-2 following BMT in a murine model for acute myeloid leukemia in SJL/J mice. *Exp Hematol* 23:196-201
Weiss L, R. S., Slavin S. 1995. The role of antibodies to IL-2 receptor and Asialo GM1 on graft-versus-leukemia effects induced by bone marrow allografts in murine B cell leukemia. *Bone Marrow Transplant* 16:457-461
Weiss L, R. S., Slavin S. 1999. Allogeneic cell therapy in murine B-cell leukemia (BCL1): 2. The role of non-activated and rIL-2-activated CD4+ and CD8+ T cells in immunotherapy for leukemia. *Cytokines Cell Mol Ther* 5:153-158
Xun CQ, T. J., Jennings CD, Brow. 1995. The effect of human IL-2-activated natural killer and T cells on graft-versus-host disease and graft-versus-leukemia in SCID mice bearing human leukemic cells. *Transplantation* 60:821-827

## Patentansprüche

1. Antigen präsentierende Zelle erhältlich aus Prä-zellen, ausgewählt aus der Gruppe bestehend aus Stammzellen, Monozyten, DCs, CD34+ Stammzellen, CD34+ Progenitor-Stammzellen, B-Zellen eines mit einem allogenen Knochenmarkstransplantat, welches auch Blutlymphozyten enthalten kann, zu behandelnden Patienten und/oder aus bereits vorhandenen Antigen präsentierenden Zellen (APC), wobei die Prä-zellen oder die bereits vorhandenen (APC)
- einer Behandlung mit Bindemolekülen gegen B7 (1 und/oder 2) und/oder CD83 und/oder CD40 und/oder CTLA4 und/oder Fas und/oder den TRAIL-Rezeptor auf T-Zellen unterworfen werden, und diese Prä-zellen oder APC in vitro mit allogenen Lymphozyten eines fremden Stammzell-Transplantatspenders inkubiert werden,
- und/oder ganz oder teilweise unreife dendritische Zellen (DC; z.B. in vitro abgeleitete) verwendet werden und diese in vitro mit allogenen Lymphozyten eines fremden Stammzell-Transplantatspenders inkubiert werden,
- und/oder die genannten Zellen mit mindestens einem Gen für ein Fas-Bindemolekül transfiziert und diese in vitro mit allogenen Lymphozyten eines fremden Stammzell-Transplantatspenders inkubiert werden,
- und/oder die genannten Zellen mit mindestens einem Gen für einen Tumor-Nekrosis-Faktor-related apoptosis-inducing ligand (TRAIL)-Rezeptor-Bindemolekül zu transfiziert und diese in vitro mit allogenen Lymphozyten inklusive von Blutlymphozyten eines fremden Stammzell-Transplantatspenders inkubiert werden,
- und/oder die genannten Zellen durch Zugabe von Aspirin, 1a,25 Dihydroxyvitamin D3, 1a,25(OH)2-16-ene-23-yne-26,27-hexafluoro-19-nor-vitamin D3 (D3 analog), Vitamin D3, Glucocorticoide dendritische Zellen in einem Zustand gehalten werden, der die immunstimmulatorischen Eigenschaften der dendritischen Zellen einschränkt und diese in vitro mit allogenen Lymphozyten eines fremden Stammzell-Transplantatspenders inkubiert werden,
- und/oder die genannten Zellen mit mindestens einer genetischen Sequenz für Interleukin 10 (IL-10) transfiziert und diese in vitro mit allogenen Lymphozyten eines fremden Stammzell-Transplantatspenders inkubiert werden,
- und/oder die genannten Zellen durch Zugabe von Interleukin 10 an der Aktivierung von T-Zellen gehindert werden und diese in vitro mit allogenen Lymphozyten eines fremden Stammzell-Transplantatspenders inkubiert werden,
- und/oder die genannten Zellen durch Zugabe von Interferon a oder g oder b (IFNa oder IFNg oder IFNb) zur Synthese von Tumor-Nekrosis-Faktor-related apoptosis-inducing ligand (TRAIL) veranlaßt und diese in vitro mit allogenen Lymphozyten eines fremden Stammzell-Transplantatspenders inkubiert werden,
- und/oder die genannten Zellen mit mindestens einem Gen für CTLA4-Bindemoleküle transfiziert und diese in vitro mit allogenen Lymphozyten eines fremden Stammzell-Transplantatspenders inkubiert werden,
- und/oder die genannten Zellen mit mindestens einem Gen für CD83-Bindemoleküle und gegebenenfalls für CD40-Bindemoleküle und gegebenenfalls für B7 (CD80/CD86)-Bindemoleküle transfiziert und diese in vitro mit allogenen Lymphozyten eines fremden Stammzell-Transplantatspenders inkubiert werden.

2. Zelle gemäß Anspruch 1, wobei die Zelle aus dem Empfänger des Transplantates stammen und bei Krebskranken, insbesondere solchen mit Leukämien, Krebszellen enthalten können, vor der Inkubation mit dem Transplantat so behandelt werden, dass eine Proliferation dieser Zellen ausbleibt, insbesondere durch Bestrahlung dieser Zellen.

3. Zelle nach einem der Ansprüche 1 und/oder 2, **dadurch gekennzeichnet, dass** eine Transfektion der Prä-zellen (Stammzellen, Monozyten, DCs, CD34+ Stammzellen, CD34+ Progenitor-Stammzellen und/oder B-Zellen) oder APC eine Expression von Fas-Bindemolekülen und/oder TRAIL-Rezeptor-Bindemolekülen und/oder IL-10 und/oder B7-Bindemolekülen und/oder CTLA4-Bindemolekülen und/oder CD83-Bindemolekülen und/oder CD40-Bindemolekülen bewirkt.

4. Zelle nach mindestens einem der Ansprüche 1 bis 3 enthaltend Nukleinsäuren, die Fas-Bindemoleküle und/oder TRAIL-Rezeptor-Bindemoleküle und/oder IL-10 und/oder B7-Bindemoleküle und/oder CTLA4-Bindemoleküle und/oder CD83-Bindemoleküle und/oder CD40-Bindemoleküle kodieren.

5. Zelle nach mindestens einem der Ansprüche 1 bis 4, bei denen die CD83 bindenden Moleküle vorzugsweise Antikörper, monoklonale Antikörper, F(ab)_{2,} Einzelkettenantikörper (scFv), und/oder F_{ab}-Fragmente sind.

6. Zelle nach mindestens einem der Ansprüche 1 bis 5, wobei die Proteine, die affine Strukturen zu B7-Rezeptoren aufweisen CTLA4, CTLA4-Abkömmlinge (z.B. CTLA4Ig), CD28, Antikörper, F(ab)_{2,} scFv und/oder F_{ab}-Fragmente sind.

7. Erfindungsgemäße Zelle nach mindestens einem der Ansprüche 1 bis 6, wobei die Proteine, die affine Strukturen zu CD40-Rezeptoren aufweisen CD40L, CD40L-Abkömmlinge, Antikörper, F(ab)_{2,} scFv und/oder F_{ab}-Fragmente sind.

8. Erfindungsgemäße Zelle nach mindestens einem der Ansprüche 1 bis 7, wobei die Proteine, die affine Strukturen zu CTLA4 (CD152)-Rezeptoren aufweisen Antikörper, F(ab)_{2,} scFv und/oder F_{ab}-Fragmente sind.

9. Zelle nach mindestens einem der Ansprüche 1 bis 8, wobei die Proteine, die affine Strukturen zu Fas-Rezeptoren aufweisen FasL, FasL-Abkömmlinge, Antikörper, F(ab)_{2,} scFv und/oder F_{ab}-Fragmente sind.

10. Zelle nach mindestens einem der Ansprüche 1 bis 9, wobei die Proteine, die affine Strukturen zum TRAIL-Rezeptor aufweisen TRAIL, TRAIL-Abkömmlinge, Antikörper, F(ab)_{2,} scFv und/oder F_{ab}-Fragmente sind.

11. Zelle nach mindestens einem der Ansprüche 1 bis 10, **dadurch gekennzeichnet, dass** die Nukleinsäuren DNA, RNA, Oligonukleotide, Polynukleotide, Ribozyme, Peptidnukleinsäuren (PNA) sind.

12. Zelle nach mindestens einem der Ansprüche 1 bis 11, **dadurch gekennzeichnet, dass** die DNA Regulationselemente wie Enhancer, Promotoren, polyA-kodierende 3'-Enden zur Transkription der DNA in RNA enthält, die RNA Regulationselemente zur Translation der RNA in Protein enthält.

13. Zelle nach mindestens einem der Ansprüche 1 bis 12, wobei die Nukleinsäuren für eine Signalsequenz kodieren oder die Expressionsprodukte eine Signalsequenz besitzen, die den Transport der Expressionsprodukte zur Plasmamembran bewirkt.

14. Zelle nach mindestens einem der Ansprüche 1 bis 13, wobei die Nukleinsäuren für eine Signalsequenz kodieren oder die Expressionsprodukte eine Signalsequenz besitzen, die den Verbleib der Expressionsprodukte im endoplasmatischen Retikulum, dem Golgi-Apparat, dem Trans-Golgi-Netzwerk oder intrazellulären Vesikeln bewirkt.

15. Verfahren zur Herstellung einer Zelle gemäß einem der Ansprüche 1 bis 14, aus Stammzellen, Monozyten, DCs, CD34+ Stammzellen, CD34+ Progenitor-Stammzellen und/oder B-Zellen eines mit einem Knochenmarkstransplantat zu behandelnden Patienten Antigen-präsentierende Zellen
- zu generieren, die einer Behandlung mit Bindemolekülen gegen B7, CD83, CD40, CTLA4, Fas, den TRAIL-Rezeptor auf T-Zellen oder Kombinationen davon unterliegen, und die DC in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch die Blockade der Costimulation durch die dendritischen Zellen abzuschalten und/oder sie in den programmierten Zelltod (Apoptose) zu zwingen und/oder sie zu regulatorischen T-Zellen zu differenzieren,
- und/oder ganz oder teilweise unreife dendritische Zellen zu verwenden und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch die bei unreifen DCs fehlende Costimulation abzuschalten und/oder sie in den programmierten Zelltod (Apoptose) zu zwingen und/oder sie zu regulatorischen T-Zellen zu differenzieren,
- und/oder die genannten Zellen - mit mindestens einem Gen für ein Fas-Bindemolekül zu transfizieren und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch den von den dendritischen Zellen an der Plasmamembran exprimierten FasL in den programmierten Zelltod (Apoptose) zu zwingen,
- und/oder die genannten Zellen mit mindestens einem Gen für den (z.B. humanen) Tumor-Nekrosis-Faktor-related apoptosis-inducing ligand (TRAIL)-Rezeptor-Bindemolekül zu transfizieren und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch das von den dendritischen Zellen an der Plasmamembran exprimierte TRAIL-Rezeptor-Bindemolekül in den programmierten Zelltod (Apoptose) zu zwingen,
- und/oder die genannten Zellen durch Zugabe von Aspirin, 1a,25 Dihydroxyvitamin D3, 1a,25(OH)2-16-ene-23-yne-26,27-hexafluoro-19-nor-vitamin D3, Vitamin D3, Glucocorticoide dendritische Zellen in einem Zustand zu halten, der die immunstimmulatorischen Eigenschaften der dendritischen Zellen einschränkt und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch die reduzierte Costimulation abzuschalten und/oder sie in den programmierten Zelltod (Apoptose) zu zwingen und/oder sie zu regulatorischen T-Zellen zu differenzieren,
- und/oder die genannten Zellen mit mindestens einem Gen für Interleukin 10 (IL-10) transfiziert, welches die immunstimmulatorischen Eigenschaften der dendritischen Zellen einschränkt und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, abzuschalten und/oder sie in den programmierten Zelltod (Apoptose) zu zwingen und/oder sie zu regulatorischen T-Zellen zu differenzieren,
- und/oder die genannten Zellen mit Interleukin 10 versetzt, welches die immunstimmulatorischen Eigenschaften der dendritischen Zellen einschränkt und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, abzuschalten und/oder sie in den programmierten Zelltod (Apoptose) zu zwingen und/oder sie zu regulatorischen T-Zellen zu differenzieren,
- und/oder die genannten Zellen mit mindestens einem Gen für CTLA4-Bindemoleküle zu transfizieren und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsiorissysteme und TCR/MHC-Komplexe intensive Wechselwirkung mit den dendritischen Zellen eingehen, durch das von den dendritischen Zellen an der Plasmamembran exprimierte CTLA4-Bindemolekül abzuschalten und/oder sie in den programmierten Zelltod (Apoptose) zu zwingen und/oder sie zu regulatorischen T-Zellen zu differenzieren,
- und/oder die genannten Zellen mit mindestens einem Gen für CD83-Bindemoleküle und/oder für CD40-Bindemoleküle und/oder für B7 (CD80/CD86)-Bindemoleküle zu transfizieren und diese in vitro mit allogenen Stammzellen oder mit allogenen T-Zellen oder mit allogenen Lymphozyten eines fremden Stammzell-Transplantatspenders zu inkubieren, um die alloreaktiven T-Zellen, die über komplexe Adhäsionssysteme und TCR/MHC-Komplexe intensive Wechselwirkung mit den dendritischen Zellen eingehen, durch das von den dendritischen Zellen im endoplasmatischen Retikulum und/oder Golgi und/oder an der Plasmamembran und/oder extrazellulär exprimierte CD83- und/oder CD40-Bindemolekül und/oder B7-Bindemolekül, welches das CD83 und/oder CD40 und/oder B7 der dendritischen Zellen bindet, abzuschalten und/oder sie in den programmierten Zelltod (Apoptose) zu zwingen und/oder sie zu regulatorischen T-Zellen zu differenzieren,
- die durch eine dieser Prozeduren und/oder eine beliebige Abfolge dieser Prozeduren positiv selektierten nicht alloreaktiven, allologen T Zellen werden dem Patienten mit den allologen Stammzellen infundiert, vorzugsweise ins Knochenmark, um einen Graft-versus-Leukämie-Effekt (GvL) zu erhalten.

16. Verfahren nach Anspruch 15, wobei die genannten Zellen ex vivo durch Behandlung mit Viren, viralen Vektoren, bakteriellen Vektoren, Plasmiden, die durch viralen Gentransfer, Elektroporationstechniken, Iontophorese, ballistischen Methoden und/oder anderen Techniken zur Einschleusung von Molekülen in eukaryotische Zellen transfiziert wird.

17. Verfahren nach Anspruch 15 und/oder 16, wobei eine genannte Zelle durch Behandlung mit Viren, viralen Vektoren, bakteriellen Vektoren, Plasmiden die durch viralen Gentransfer, Elektroporationstechniken, Iontophorese, ballistischen Methoden und/oder anderen Techniken zur Einschleusung von Molekülen in eine Zelle mit erhöhter Produktion von Fas-Bindemolekül und/oder TRAIL-Rezeptor-Bindemolekül und/oder CD83-Bindemoleküle, CD40-Bindemoleküle, B7 (CD80/CD86)-Bindemoleküle, CTLA4-Bindemolekül, IL-10 transfiziert wird, wodurch T-Zellen, welche über allologe Antigene, die z.B. an MHC-Molekülen präsentiert werden, an die Antigen-präsentierende Zelle binden, ausgeschaltet werden.

18. Verfahren nach mindestens einem der Ansprüche 15 bis 17, wobei Moleküle wie Antikörper, Proteine, Peptide, Peptidomimetica, CTLA4, CTLA4-Derivate wie CTLA4Ig, CD28, CD40L und/oder Bestandteile und/oder Kombinationen dieser Moleküle, die z.B. B7-1, B7-2, CD83, CD40 binden, welche eine in Gegenwart einer Alloantigenpräsentation stattfindende Stimulation und/oder Co-Stimulation von T-Zellen behindert, die mit der erfindungsgemäßen Zelle in Kontakt gebracht werden.

19. Verfahren nach mindestens einem der Ansprüche 15 bis 16, wobei die Moleküle (Fas-Bindemolekül, TRAIL-Rezeptor-Bindemolekül, CD83-Bindemoleküle, CD40-Bindemoleküle, B7 (CD80/CD86)-Bindemoleküle, CTLA4-Bindemolekül, IL-10-Gen) gemäß Anspruch 12 durch Vehikel, wie Liposomen, Hydrogele, Zyklodextrine, Nanokapseln, Nanopartikel, bio-adhäsive Mikrokugeln und/oder durch Elektroporationstechniken, Iontophorese, ballistische Methoden und/oder andere Techniken zur Einschleusung von Molekülen in die erfindungsgemäße Zelle transferiert werden.

20. Verfahren nach mindestens einem der Ansprüche 15 bis 19, wobei Nukleinsäuren durch Viren, virale Vektoren, bakterielle Vektoren, Plasmide die durch viralen Gentransfer, Elektroporationstechniken, Iontophorese, ballistische Methoden und/oder andere Techniken zur Einschleusung von Molekülen in die genannten Zellen transferiert werden.

21. Prä-zelle ausgewählt aus der Gruppe bestehend aus Stammzellen, Monozyten, DCs, CD34+ Stammzellen, CD34+ Progenitor-Stammzellen und/oder B-Zellen, **dadurch gekennzeichnet, dass** ein Inhibitor der Hypusinbiosynthese, wie z.B. GC7 (N¹ -guanyl-1,7-diaminoheptane) verwendet wird, um die Produktion von funktionalem eIF-5A zu behindern, was wiederum die CD83-Biosynthese behindert.

22. Arzneimittel enthaltend mindestens eine erfindungsgemäße Zelle oder nach mindestens einem der Ansprüche 1 bis 14.

23. Arzneimittel nach Anspruch 22, wobei mindestens eine erfindungsgemäße Zelle zur ex vivo Applikation formuliert ist.

24. Verwendung mindestens einer erfindungsgemäßen Zelle nach mindestens einem der Ansprüche 1 bis 14 zur Herstellung eines Arzneimittels zur Behandlung von Immunreaktionen gegen allologe Gewebsmerkmale.

25. Verwendung nach Anspruch 24, wobei die zu behandelnden Immunreaktionen in Verbindung mit allologen Gewebsmerkmalen, deren Gensequenzen und/oder Teilsequenzen, insbesondere Haupthistokompatibilitätskomplexe, MHC I, MHC II, Rhesus Faktor, Nebenhistokompatibilitätsantigene (minor histocompatibility antigens) steht.

26. Verwendung nach Anspruch 1 bis 25, wobei die Bindemoleküle in einer bevorzugten Ausführung vernetzt werden, was durch andere Bindemoleküle, wie z.B. Antikörper, geschehen kann, die wiederum die Bindemoleküle gegen CD83, CD80, CD86, CD40, CTLA4, Fas, TRAIL-Rezeptoren binden.

27. Verwendung nach Anspruch 1 bis 26, wobei die *ex vivo* selektierten Zellen dem Patienten infundiert werden und dieser danach einer *in vivo* Behandlung mit IL-2 unterliegt.

28. Verwendung nach Anspruch 1 bis 26, wobei die *ex vivo* selektierten Zellen *ex vivo* mit IL-2 oder anderen Stimulanzien, wie Phytohemaglutinin (PHA) inkubiert und danach dem Patienten infundiert werden.
